# EUROPÄISCHE PATENTSCHRIFT

(11) **EP 1 339 502 B1**
(45) Veröffentlichungstag und Bekanntmachung des Hinweises auf die Patenterteilung: **29.09.2004**
(21) Anmeldenummer: 01999436.7
(22) Anmeldetag: 04.12.2001
(51) Int. Cl.: B05C 9/14, A61L 27/54, A61L 27/28, B05D 3/06, A61F 2/00

(54) **Vorrichtung zur Beschichtung eines Gegenstands mit einem Wirkstoff und Verfahren zur Herstellung eines partiellen oder vollständigen Wirkstoffüberzuges auf und im Implantaten und Onplantaten sowie Verwendung dieses Verfahrens und durch dieses Verfahren hergestelltes Erzeugnis**
Device for Coating an Object with an Active Ingredient and Method for Producing a Partial or Complete Active Ingredient Coating on and in Implants and Onplants as well as Use of or Product Obtained through such a Method
Dispositif de revêtement d'un objet par un principe actif et procédé de production d'un revêtement de principe actif partiel ou total sur et dans des implants à introduire ou apposer, ainsi que utilisation de et produit obtenu par cette méthode

(30) Priorität: 04.12.2000 DE 10060354
(43) Veröffentlichungstag der Anmeldung: 03.09.2003
(73) Patentinhaber: Grüssner, Uwe Emil, 65195 Wiesbaden (DE)
(72) Erfinder: BAUER, Jörg, 64293 Darmstadt (DE); GRÜSSNER, Uwe, Emil, 65195 Wiesbaden (DE)
(74) Vertreter: Kampfenkel, Klaus, Dipl.-Ing.DE)
(86) Internationale Anmeldenummer: PCT/EP2001/014157
(87) Internationale Veröffentlichungsnummer: WO 2002/045867

(56) Entgegenhaltungen:
- DE-A- 19 918 435

## Beschreibung

Die Erfindung betrifft ein Verfahren und eine Vorrichtung zur Beschichtung eines Gegenstands mit einem Wirkstoff.

Die medizinische Technologie hat in den letzten Jahrzehnten erstaunliche Erfolge verzeichnet. Dies resultiert daraus, daß zum einen differenziertere Behandlungsmethoden, zum anderen die gerätemäßige Ausstattung in medizinischen Institutionen - Kliniken, universitären Einrichtungen - bzw. im niedergelassenen Bereich nachgewiesenermaßen eine weitaus höhere Qualität erhalten hat. Verbesserungen lassen sich dadurch häufig nur noch durch hochinnovative methodische oder gerätemäßige Verfahren erzielen.

In dem vorliegendem Fall können durch die erfindungsgemäße neuartige Beschickungsmethode Fertigprodukte oder Halbzeuge der Produktgruppe der Medizinprodukte oder der Arzneimittel oder artnahe Produkte derart ergänzt werden, daß die Wirkung nicht mehr ausschließlich dem ursprünglichen Verwendungszweck dient, sondern die zusätzlichen Funktion einer lokalen Wirkstoff- bzw. Substanzeinbringung hat: hierdurch können Wirkungen erzielt werden, die beispielsweise der Verringerung des Allergisierungs- und Entzündungspotentiales oder der Minimierung immunologischer Prozesse, der Förderung regenerativer Prozesse, der Infektionsabwehr und Kontaminationsbekämpfung dienen, oder in Kombination untereinander auftreten.

Aufgrund der Vielfalt der Möglichkeiten soll nachfolgend die Methodik der Erfindung am Beispiel von Infektionen dargelegt werden. Dies gilt jedoch nur beispielhaft für alle anderen Wirkungen.

Postoperative Infektionen konnten durch die Standardisierung der OP-Methoden und der verwendeten Materialien bei Standardoperationen auf Infektionsraten um 1% gesenkt werden. Ein großer Teil dieser Infektionen kann nicht durch die körpereigenen Abwehrmechanismen korrigiert werden. Hierzu benötigt der Organismus Antibiotika, die aufgrund der Unwägbarkeiten standardmäßig vor, während und nach der Operation gegeben werden.

Mit dem Wachsen der Ersatzteilchirurgie ist nun eine neue Dimension in die Qualität von Infektionsrisiken entstanden. Ein durch eine Körperöffnung eingeführtes Implantat oder Onplantat kann direkt auf seiner Oberfläche Infektionsrisiken beherbergen, die einmal implantiert einen idealen Nährboden für seine Ausdehnung erhalten. Selbst die inzwischen als optimal zu bezeichnenden mikrobiologischen Zustände in den aseptischen Operationsräumen, zumindest in den größeren Industrieländern, mindern nicht ein gewisses Risiko der Infektion bedingt durch die Operation an sich, das verwendete Material und die beteiligten Personen.

Aus diesen Gründen werden verschiedene Methoden zur Infektionsprophylaxe angewendet. So werden z.B. antibakterielle Wirkstoffe injiziert, bzw. über den Magen oder die Blutbahnen zugeführt. In der Regel werden die antibakteriellen Wirkstoffe jedoch in einem weiten Gebiet im Organismus verteilt und nicht an dem Ort, wo sie benötigt werden. Zusätzlich haben allgemein verteilte Antibiotika den gravierenden Nachteil, daß sie Nebenwirkungen hervorrufen, die unerwünscht und oftmals schädlich sind.

So ist das Ziel der Forschung und Entwicklung neue Antibiotikaapplikationen zu entwickeln, die es ermöglicht den Wirkstoff dort, wo er benötigt wird, zu plazieren und die Dosierung der lokal applizierten Wirkstoffmenge auf ein sicheres Mindestmaß zu limitieren.

Eine Richtung ist die Entwicklung von Trägermaterialien, die einen Wirkstoff vor Ort freigeben. Diese Freigabe erfolgt in der Regel über ein bestimmtes Zeitmaß hinweg mit unterschiedlichen Mengenverteilungen über den Abgabezeitraum. Hierbei werden Hilfsstoffe, an die der Wirkstoff gebunden ist, in die Operationswunde mit hineinimplantiert, um dort ihre Wirkung, der Bekämpfung von Infektionen und der Vorbeugung der Entstehung von Infektionen, zu entfalten.

So ist beispielsweise aus der dem Oberbegriff des Vorrichtungsanspruchs 1 bzw. dem Oberbegriff des Verfahrensanspruchs 9 entsprechenden DE 199 18 435 A1 ein Verfahren zur Herstellung eines Überzugs- und Bindemittels für orale oder dermale Arzneiformen bekannt. Das Überzugs- und Bindemittel, welches keinen Wirkstoff enthält, besteht aus Copolymer, einem Weichmacher und einem Emulgator.

In einer weiteren Gruppe von Infektionen sind diejenigen Produkte zu integrieren, bei denen ein Implantat durch den Körper mit der Außenwelt verbunden ist, wie z.B. bei den externen Fixateuren. Hier ist der Infektionsort meist die Durchtrittstelle durch die Haut des Patienten. Selbst hohe und häufige Hygienemaßnahmen helfen hier oftmals nicht, so daß zusätzlich therapiert werden muß.

Insgesamt betrachtet sind die Maßnahmen zur Bekämpfung von Infektionen und die Prophylaxe gegen Infektionen nach dem heutigen Stand der Medizin ein Muß. Da die derzeitigen Methoden die Gefahr unerwünschter Nebenwirkungen beinhalten, ist die Entwicklung von ortsgebundenen Wirkstoffverabreichungen eine technologische Herausforderung.

Erfindungsgemäß wurde nun sowohl eine Vorrichtung gemäß Anspruch 1 als auch ein Verfahren gemäß Anspruch 9 entwickelt, mit dem die vorab beschriebenen Nachteile der Therapiemöglichkeiten ausgeräumt werden, und eine lokale Therapie am Wirkort durchgeführt werden kann, ohne sekundäre Implantate einzuführen oder ohne eine zusätzliche Maßnahme wie Spritze oder Tablette zu benötigen. Dabei ist die Verwendung solcher erfindungsgemäß beschickter Materialien in der stationären oder ambulanten Behandlung möglich.

Die Aufgabe wird dadurch gelöst, daß auf die inneren und/oder äußeren Oberflächen eines zu implantierenden / onplantierenden Implantates / Onplantates ein Wirkstoff in der Form aufgebracht wird, daß die mit Wirkstoff versehene Oberfläche im wesentlichen vollständig mit diesem Wirkstoff in einer vollständig geschlossenen Schicht oder teilweise mit wirkstoffrelevanten Abständen, die vom Diffusionsverhalten des Wirkstoffes abhängig sind, versehen ist. Dabei kann der Wirkstoff auf dem Teil nur Segmente der Oberfläche bedecken oder dieses ganz umschließen. Bei der segmentierten Beschichtung ist eine ein- oder mehrseitige Beschichtung erfindungsgemäß möglich, wobei diese wiederum geschlossen oder segmentiert sein kann. Erfindungsgemäß ist die Wirkstoffschicht wahlweise eine feste Schicht, eine elastische Schicht oder eine weiche Schicht, wobei eine Graduierung bzw. Schichtung unterschiedlicher Viskositäten der Wirkstoffschicht erfindungsgemäße Variationen darstellen.

Neben dieser Härteklassierung besteht die Möglichkeit unterschiedliche Wirkstoffe an unterschiedlichen Stellen des Teiles aufzubringen, so daß erfindungsgemäß eine Beschichtung mit lokal unterschiedlichen Wirkstoffen entstehen kann, oder daß Kombinationen aus einem oder mehreren Wirkstoffen sowohl in der Härte unterschiedlich als auch in der Zusammensetzung unterschiedlich entstehen können.

Erfindungsgemäß kann auch eine Beschichtung mit Wirkstoff entstehen, die eine graduierte Freisetzungsrate des oder der Wirkstoffe beinhaltet, so daß im erfindungsgemäßen Beschichtungsverfahren eine große Varianz mit vielen gestalterischen Freiheitsgraden resultiert.
Wesentlich ist dabei die Festigkeit des Wirkstoffes an der Oberfläche des Teiles. Durch das erfindungsgemäß beschichtete Teil wird der Wirkstoff in die Wunde eingeführt oder auf diese aufgelegt, ohne daß signifikante Mengen Wirkstoff durch Reibung oder Abschabungen außerhalb des Wirkortes anfallen können. Im klassischen Fall werden z.B. metallische Endoprothesen mittels eines Schlagwerkzeuges in das vorbereitete Knochenbett eingeschlagen. Die entstehende Presspassung ist für das Anwachsen der Endoprothese unabdingbar.

Werden durch Kontamination der Prothese oder des Werkzeuges oder sonstigem Operationszubehör Bakterien in die Wunde eingebracht, so ist das Implantat und das Knochengewebe ohne Schutz. Die Operation kann durchaus bis zu einer Stunde und mehr betragen. Die Gefahr einer zusätzlichen Baktierieneinschleppung wächst hierdurch. Ist nun die Endoprothese mit einem prophylaktischem Schutz versehen, so kann ein eingebrachter Infektionsherd direkt vor Ort bekämpft und die ungehinderte Einheilung des Implantates gewährleistet werden. Prothesenlockerungen durch Infektionsgebiete mit ihren erhöhten Zellaktivitäten können nicht mehr auftreten.

Andererseits entsteht bei der Einbringung der Prothese in das Knochenbett durchaus eine nicht unerhebliche schabende Reibung auf der Oberfläche des Implantates, was eine feste Haftung des Wirkstoffes auf der Implantatoberfläche notwendig macht, denn auch die Prothesenspitze oder deren Kanten und Bögen muß oder kann prophylaktisch geschützt sein.

Im Falle einer Verplattung einer Defektstelle ist es Stand der Technik eine metallische Platte überbrückend auf den Defekt aufzulegen und diese mittels spezieller Schrauben zu fixieren. Der gefährdetste Bereich sind dabei die Plattenunterseite und die durch die Schrauben enstehenden Kavitäten. Können diese nun mit einem prophylaktischen Wirkstoffschutz versehen werden, so reduziert sich die Gefahr einer Lockerung, und mit der Reduzierung der Lockerungsrate erhöht sich der positive Genesungsverlauf.

In einer weiteren Anforderung treten Implantate durch die Haut nach außen und erfüllen in der inneren und äußeren Konstruktion ihre Funktion. Dies ist vor allem bei Fixateuren, bei den sogenannten Kirschnerdrähten, bei Streckund Dehnvorrichtungen u.a. der Fall. Hier ist die größte Gefahrenstelle, neben der Infektionsgefahr durch die Operation selbst, die Infektion durch die Durchtrittsstelle, welche entlang des Implantatkanals in die Wunde hineinwandern kann. Ein harter Schutz durch einen Wirkstoff stellt hier ein signifikant besseres Produkt für den Patienten dar als die herkömmlichen medikamentösen Behandlungen.

Gemeinsam ist diesen vorangestellten als Beispiele zu verstehende Einsatzmöglichkeiten, Prothesen und Implantatbeispielen die Elimination der Fehlermöglichkeit durch Deplazierung des Wirkstoffes und die Reduzierung der Gefahr der persönlichen Fehler durch Unter- bzw. Überdosierung.

Diese Modelle lassen sich auf alle metallischen Implantate, Onplantate und erfindungsgemäß auch auf die verwendeten Instrumentarien übertragen. Die Instrumente sind noch von untergeordneter Bedeutung, aber bei der Zunahme an hochsensiblen Operationen oder bei Operationen ohne optimale OP-Raum-Umgebung, wie z.B. bei Notoperationen an Unfallstellen oder in Ländern geringer hygienischer Standards sind diese in den nächsten Jahren sicherlich von wachsender Bedeutung.

Auch der passive Schutz von Instrumenten gegen Besiedelung durch eine derartige Beschichtung kann sinnvoll sein, wenn z.B. Notoperationsbestecke längere Lagerzeiten überstehen sollen.

Unbeschadet sind in einem weiteren Anwendungsgebiet andere als die metallischen Materialien zu betrachten. So ist der Schutz von Patienten vor Infektionen sicherlich auch abhängig, wenn auch nicht mit einer so hohen Wertigkeit wie bei Implantaten, von der Reinheit der untergeordneten Hilfsstoffe. Der Schutz mit Wirkstoff kann hier zumindest mögliche Gefährdungen der peripheren Bereiche und der vom Patienten ersichtlichen Partien gemindert werden.

Die Heilung eines Schnittes der einer Versorgung unterlag, ist sicherlich unkritisch. Jedoch ist in der Regel die Narbe bei Auftreten einer Entzündung im Endeffekt weniger "schön", d.h. im Narbenbereich wulstiger und hervorgehobener, als ohne Entzündung. Werden nun erfindungsgemäß die Verschlußmittel einer solchen Wunde, sei es durch einen Eingriff hervorgerufen, oder als Platzwunde vorliegend, oder sonstwie entstanden, mit einem durch eine erfindungsgemäße Wirkstoffschicht geschütztem Material durchgeführt, so kann die Infektionswahrscheinlichkeit herabgesetzt werden. Als Materialien kommen hier vor allem die chirurgischen Fäden und die verwendeten Nadeln, die Klammern der Klammergeräte und andere in Frage.

Besonders häufig treten Entzündungen durch die Verwendung von Injektionsapparaturen auf. Diese Problematik ist insbesondere durch die Übertragung von AIDS in die Diskussion und das Verständnis gelangt. Aber auch in Krisengebieten oder bei Umweltkatastrophen ist diese Problematik nicht zu vernachlässigen. Werden nun diese Injektionsnadeln mit einem prophylaktischen Wirkstoffschutz in einer z.B. harten Variante mit einem Wirkstoff beschichtet, so lassen sich diese Problematiken sicherlich in vielen Fällen reduzieren.

Werden in einer besonderen Anwendung temporär Geräte verwendet, so ist deren Schutz auch temporär möglich. In diesem speziellen Fall ist z.B. an die Träger von Hörgeräten gedacht, die öfters mit entzündlichen Reaktionen an den Kontaktstellen im Ohr zu kämpfen haben. Wird nun der kontaktierende Bereich eines solchen Hörgerätes für einen solchen Fall mit einem Wirkstoffschutz versehen, der eine gewisse Materialhärte besitzt, so können unter Umständen die verwendeten Salben, die das Gerät verschmieren und zum Teil auch die Hörqualität herabsetzen, ersetzt werden. Die konzentrierte Behandlung der Entzündung ist nun auch während des Tragens der Hörgeräte möglich.

In einem weiteren Fall ist die Beschichtung von Sehhilfen erfindungsgemäß vorgesehen. Hierbei werden insbesondere die abstützenden Bauteile der Sehhilfe im nasalen Bereich und die Haltebügel über den Ohren mit einer Wirkstoffschicht beschichtet, so daß selbst bei vorliegender Entzündung eine Behandlung möglich ist, ohne auf die Sehhilfe verzichten zu müssen.

Ein weiterer erfindungsgemäße Anwendungsfall kann die prophylaktische Wirkstoffversorgung aktiver Bauelemente darstellen. So ist ein Schutz von Pumpen, Dosiergeräten, Signalgeräten, Impulsgebern oder analytischen Implantaten wie Insulindetektoren und andere durchaus realistisch.

In der neueren Praxis stellen sich aber auch die dentalen Implantate als geeignete Verfahrenspartner dar. So ist ein mit einem derartigen Wirkstoff beschichtetes Kieferimpalantat, oder Dentalimplantat, weit weniger gefährdet als ein ungeschütztes.

Betrachtet man in diesem neuen medizinischen Feld die Methoden der Operationen und der eingesetzten Materialien, so ist die Verwendung von synthetischen Sehnen und Bändern und Knorpelersatz ein durchaus wachsender Anwendungsmarkt von extrem steigender Bedeutung. Erfindungsgemäß können nun mit diesem Verfahren auch diese Materialien mit einem Wirkstoffschutz versehen werden. Dabei kann der Wirkstoff nur auf der äußeren Oberfläche aufgebracht werden, oder aber auch im Inneren der Teile plaziert werden. Durch die spezielle Verarbeitungsmethode kann der Wirkstoff dabei auf die Beweglichkeit des Teiles abgestimmt werden, so daß keine funktionelle Einschränkung stattfinden kann.

Insbesondere sind die Möglichkeiten der Wirkstoffbeschichtung durch das Verfahren dahingehend geeignet spezifische Werkstückeigenschaften in Hinblick auf Porosität, Flexibilität, Oberflächenstruktur und andere zu erhalten. Da die Beschichtung ohne Zuhilfenahme von Trägersubstanzen erfolgt ist das aufgebrachte Volumen zur Wirkstoffmengenfreisetzung stets geringer als bisher bekannt, so daß das implantierte Fremdvolumen eine minimale Ausdehnung hat. Fremdkörperreaktionen aufgrund des Wirkstoffes sind daher stets minimalisiert. Anders ist dies bei trägergebundenen Wirkstoffen, die stets mit der Problematik der Fremdkörperreaktion belastet sind. Auch ist durch die Monomaterialgabe eine zusätzliche Zellaktivität, hervorgerufen durch die Trägersubstanzen oder deren Abbauprodukte, nicht gegeben. Durch die Verringerung der zusätzlichen Zellaktivitäten wird die Wirksamkeit der Wirkstoffe nicht überschattet, so daß sie ihre optimale Wirkung entfalten können. Ebenfalls bedingt durch die trägerlose Wirkstoffbeschichtung kann die Problematik der Spaltbildungen verringert werden. Normalerweise tragen die Träger in ihrer Schichtdicke auf, was einen signifikanten Abstand zwischen Werkstückteil und z.B. Knochen hervorruft. Dieser Abstand bildet bei Lösung der Träger mit gleichzeitiger Wirkstofffreigabe eine Lücke zwischen Teil und Körperteil die ungewünschte Instabilitäten mechanischer und biomechanischer und biologischer Art verursacht. Kann dieser Spalt minimalisiert werden, so verringert sich automatisch die Gefahr des Kontaktspieles (Bewegung). Insbesondere ist durch die erfindungsgemäße Methode die Möglichkeit gegeben, eine sehr hohe Wirkstoffdosis in einer minimalen Schichtdicke einzubringen. Diese Schichtstärken sind durchaus im Bereich unterhalb des µm Maßstabes möglich, so daß Wirkstoffschichtdicken im nm Bereich realisiert werden können.

Die Methode selbst wird erfindungsgemäß dahingehend gelöst, daß eine Niedertemperaturbehandlung des Teiles mit energetischen Strahlen durchgeführt wird. Diese Methode ist erfindungsgemäß notwendig, da in der Regel die zu verwendenden Wirkstoffe extrem temperaturabhängig sind. Insgesamt kann die Methode der Erzeugung einer stabilen Wirkstoffschicht dahingehend gelöst werden, daß ein Material dahingehend vorbereitet wird, daß es in seiner Mikrooberflächenstruktur derart behandelt wird, daß es Flüssigkeiten- vorzugsweise Wasser- vollflächig aufnimmt ohne daß sich Zusammenballungen aufgrund der Oberflächenspannungen ergeben. Bedingt durch die gleichmäßige Benetzung der Teileoberfläche, hier metallische Implantate oder Onplantate, ergibt sich eine entsprechend gleichmäßige Verteilung des oder der Wirkstoffe in dem Feuchtigkeitsfilm. Randständige Erhöhungen der Flüssigkeitsdichte können vernachlässigt werden, da die resultierende Wirkstoffschicht lediglich einen Bruchteil der Dicke der Flüssigkeitsschicht erreicht.

Die erfindungsgemäße Besonderheit des Verfahrens besteht nun darin, daß die zugeführten Strahlen nur zu einem geringen Teil eine Erwärmung der Flüssigkeitsschicht bewirken. Die Zufuhr von energetischen Strahlen hat sich bei der erfindungsgemäßen Methode als besonders vorteilhaft erwiesen, da die Wirkung der Strahlen auf die Wirkstoffe selbst am geringsten ist. Lediglich die für den Wirkstoffauftrag notwendige Lösungsmittelmenge muß eliminiert werden, wobei gleichzeitig eine kristalline Strukturschichtbildung des Wirkstoffes erfolgt. Je nach Länge und Intensität der Zufuhr der elektromagnetischen Energie entsteht ein mehr oder weniger hartes Kristallisat aus reinem Wirkstoff. Bedingt durch das Verfahren können nach einer ersten Schichterzeugung eine oder mehrere weitere Schichten aus demselben Wirkstoff aufgetragen werden, welcher durch eine geringere Energiedosis weicher gestaltet wird durch eine höhere Dosis härter gestaltet wird, oder der erneute Auftrag erfolgt mit einem anderen Wirkstoff oder Mischungen von Wirkstoffen, die ebenfalls mehr oder weniger hart ausgebildet werden. Wird diese Folge praktiziert, so können unterschiedliche Wirkstoffe- oder gleiche Wirkstoffenacheinander oder in unterschiedlichen Lösungsgeschwindigkeiten von oder aus der Implantat /Onplantatoberfläche abgegeben werden. Damit ist eine gesteuerte Versorgung des Wundgebietes mit dem oder den Wirkstoffen möglich.

Erfindungsgemäß elementar ist jedoch, daß die Wirkstoffe nicht spontan gelöst werden können, wie es bei den meisten Wirkstoffen in ihrer natürlichen Konsistenz normal ist, sondern über einen Zeitraum erfolgt.

Nachfolgend ist ein Anwendungsbeispiel aufgeführt, welches sowohl das Verfahren, als auch dessen Möglichkeiten exemplarisch verdeutlicht.

Eine Hüftgelenksendoprothese aus einem Cobalt-Chrom-Molybdän Stahl mit einem Standardkopfanschluß mit einem sogenannten Eurokonus, dessen unterste Prothesenspitze poliert ist wird nach einer Grundreinigung an der Spitze mit einer temporären Folie abgedeckt. Ebenso wird der Kragen der Prothese und der Konus selbst, jedoch ohne den Prothesenhals, mit einer temporären Folie abgedeckt. Die verbleibenden Freiflächen werden nun nach klassischen Verfahren mikrooberflächenstrukturiert, z.B. durch Glasperlenstrahlen, Ätzen, Schleifen oder sonstigen Oberflächenbehandlungsmethoden. Dabei wird besonderer Wert auf die chemische Reinheit des Strahlmittels oder der Behandlungsmittel gelegt, um Oberflächenverunreinigungen zu vermeiden. Nach der Entfernung der Abdeckfolien erfolgt eine gründliche Reinigung der Hüftgelenksendoprothese um eventuelle Stahlmittelrückstände oder der Behandlungsmittel restlos zu entfernen. Dies wird in der Regel mittels Bürste und Ultraschallbäder durchgeführt. Daran anschließend wird die Hüftgelenksendoprothese vollständig entfettet und das Entfettungsmittel restlos entfernt. Die Hüftgelenksendoprothese wird in eine Halterung für die weitere Bearbeitung eingesetzt und unter überwachten Bedingungen für die Nachbehandlung an einem geeigneten Ort deponiert.

Die Oberfläche, die für die Beschichtung vorgesehen ist, wurde mathematisch bestimmt. Es ergaben sich für diesen Anwendungsfall eine Beschichtungsoberfläche von genau 80,10 cm².

In einem parallelen Arbeitsgang wird der Wirkstoff für die Beschichtung vorbereitet. Nach entsprechenden Wirkstöffuntersuchungen zur Reinheit, Aktivität und anderen Qualitätskontroll- und Sicherheitsuntersuchungen wird ein Ansatz hergestellt. Zu diesem Zweck wird in eine Vorlage aus Lösungsmittel, hier bidestilliertes steriles Wasser, eine exakt abgewogene Menge an Wirkstoff hineingegeben und homogen bis zur vollständigen Lösung oder Suspendierung des Wirkstoffes aufgelöst, bzw. suspendiert. In unserem Beispiel sind dies in 100,000 Gramm Wasser in genau 50,000 Gramm Gentamicinsulfat, dessen Aktivität mit 95 % bestimmt wurde.

Die entstandene Lösung wird temperiert- in diesem Fall bei 15 °C für die weitere Behandlung bereitgestellt.
Es ergibt sich eine Wirkstofflösung mit einem Gewicht von 150 Gramm, wovon 50,000 Gramm Gentamicinsulfat darstellen, die eine Aktivitätmenge von 47,500 Gramm aktiver Substanz darstellen.

In dem nächsten Arbeitsgang wird die Lösung des Wirkstoffes gleichmäßig auf die behandelte Oberfläche der Hüftgelenksendoprothese aufgetragen. Dies sind der Prothesenschaft ohne dessen Spitze ( die Zufuhr von Wirkstoff erfolgt in diesem Falle durch einen vor der Prothesenspitze liegenden Markraumsperre, der ebenfalls mit dieser erfindungsgemäßen Methode behandelt worden ist), der Prothesenkragen an seiner unteren Seite und der Prothesenhals ohne den anschließenden Konus. Der Auftrag erfolgt mit definierten Mengen durch ein geeignetes Gerät.

In diesem Anwendungsfall ergeben sich die nachstehenden Bilanzierungen:
Gewicht der Hüftgelenksendoprothese vor der Beschichtung 180,500 Gramm. Nach dem Auftrag der Beschichtung hat diese Prothese ein Gewicht von 181,900 Gramm. Damit ergibt sich ein Auftrag an Wirkstofflösung von 1,400 Gramm. Von diesen 1,400 Gramm aufgetragene Wirkstofflösung sind 0,4666 Gramm Gentamicinsulfat wovon 0,4433 Gramm aktive Substanz darstellen. Der Mengenrest wird von 0,9333 Gramm Wasser dargestellt.

Im nächsten Arbeitsschritt werden die so vorbereiteten mit der Wirkstofflösung versehenen Hüftgelenksendoprothesen in einer geeigneten Halterung einer Dosis an elektromagnetischen Strahlungen ausgesetzt. Die Bestrahlung erfolgt mit einer Frequenz oder mit Frequenzen, die vorzugsweise in den Frequenzbändern L - Band (0,39 GHz bis 1,55 GHz), S - Band (1,55 GHz bis 3,9 GHz), C - Band (4 GHz bis 6 GHz), X - Band (6,2 GHz bis 10,9 GHz), K -Band (10, 9 GHz bis 36 GHz), Q - Band (36 GHz bis 46 GHz) oder V - Band (46 GHz bis 56 GHz), sowie oberhalb des V - Bandes liegt bzw. liegen. Für die Beschichtung sind insbesondere die Frequenzen geeignet, bei der die Absorption in Wasser erhöht ist. Die Energiezufuhr erfolgt bevorzugt intermittierend oder gebündelt.
Der auf den Wirkstoff des Hüftgelenksimplantates entfallende Energieanteil beträgt in diesem Anwendungsfall lediglich eine geringe Leistungsarbeit.

Nach Ablauf der festgelegten Reaktionszeit wird das nunmehr beschichtete Hüftgelengsimplantat der Reaktionskammer entnommen.

Die Mengenbilanz ergibt nun ein Gewicht von 180,9610 Gramm, wovon 180,5 Gramm auf die Hüftgelenksendoprothese entfallen und 0,4610 Gramm auf die Wirkstoffmenge. Die 0,0056 Gramm Mengendifferenz sind standardisierte Verlustmengen aus Auflagepunkten und Verdunstungsmengen. Die Menge an auf der Hüftgelenksendoprothese aufgebrachten Wirkmenge beträgt demnach 0,4379 Gramm aktiver Wirksubstanz.

Umgerechnet auf die ermittelte Beschichtungsoberfläche resultiert eine Wirkstoffbeladungsmenge von 0,00547 Gramm/cm² beschichteter Fläche.
Die ermittelten Freigabeversuche in physiologischer Kochsalzlösung /Wasser / Serum ergaben eine exponentiell abfallende Freisetzungsmenge über die Zeit. Diese lag in der initialen Startphase der Freisetzung weit über den Mindestmengen der Konzentration. Ein Konzentrationsgefälle über die Umgebung konnte nicht festgestellt werden. Selbst nach längeren Lagerungszeiten in den Elutionsmedien wurden noch signifikante Wirkstoffmengen analysiert. Betrachtet man nun alle angeführten Beispiele von Implantaten oder Onplantaten oder peripheren Teilen, so kann sich der Fachmann eine ganze Reihe weiterer Anwendungsbereiche und Materialien vorstellen und aus den Dargestellten ableiten, die mit dieser Methode bearbeitet werden kann. Diese nicht näher bezeichneten anderen Anwendungsfälle werden ebenfalls erfindungsgemäß beansprucht.

In den Bereichen der Wirkstoffe, die mit diesem Verfahren verarbeitet werden können, fallen vor allem die Wirkstoffe der Gentamicine, der Clindamycine, der Vancomycine, der Penizilline und vergleichbare oder ähnliche Stoffe, wie Abmagerungsmittel/Appetitzügler, Analeptika/Antihypoxämika, Analgetika/Antirheumatika, Antiallergika, Antianämika, Antiarrhythmika, Antibiotika/Antiinfektika, Antidementiva (Nootropika), Antidiabetika, Antidota, Antiemetika/Antivertiginosa, Antiepileptika, Antihämorrhagika (Antifibrinolytika u. andere Hämostatika), Antihypertonika, Antihypoglykämika, Antihypotonika, Antikoagulantia, Antimykotika, Antiparasitäre Mittel (extern), Antiphlogistika, Antitussiva/Expektorantia, Arteriosklerosemittel, Balneotherapeutika u. Mittel zur Wärmetherapie, Betarezeptorenblocker, Calciumkanalblocker u. Hemmstoffe des Renin- Angiotensin-System, Broncholytika/Antiasthmatika, Cholagoga u. Gallenwegstherapeutika, Cholinergika, Corticoide (Internat), Dermatika, Desinfizientia/Antiseptika, Diätetika/Ernährungstherapeutika, Diagnostika u. Mittel zur Diagnosevorbereitung, Diuretika, Durchblutungsfördernde Mittel, Entwöhnungsmittel, Enzyminhibitoren, Enzympräparate u. Transportproteine, Fibrinolytika, Geriatrika, Gichtmittel, Grippemittel u. Mittel gegen Erkältungskrankheiten, Gynäkologika, Hepatika, Hypnotika/Sedativa, Hypophysen-, Hypothalamushormone, andere regulatorische Peptide u. ihre Hemmstoffe, Immunmodulatoren, Infusions- u. Standardinjektionslösungen, Organperfusionslösungen, Kardiaka, Karies-, Paradontosemittel u. andere Dentalpräparate, Koronarmittel, Laxantia, Lipidsenker, Lokalanästhetika/Neuraltherapeutika, Magen-Darm-Mittel, Migränemittel, Mineralstoffpräparate, Mund- u. Rachentherapeutika, Muskelrelaxantia, Narkosemittel, Neuropathiepräparate u. andere neurotrope Mittel, Ophthalmika, Osteoporosemittel/Calciumstoffwechselregulatoren, Otologika, Parkinsonmittel u. andere Mittel gegen extrapyramidale Störungen, Psychopharmaka, Rhinologika/Sinusitismittel, Roborantia/Tonika, Schilddrüsentherapeutika, Sera, Immunglobuline u. Impfstoffe, Sexualhormone u. ihre Hemmstoffe, Spasmolytika, Thrombozytenaggregationshemmer, Tuberkulosemittel, Umstimmungsmittel, Urologika, Venentherapeutika, Vitamine, Wundbehandlungsmittel, Zytostatika, andere antineoplastische Mittel u. Protektiva, Präparateserien/Reg. Homöopathika, Biomaterialien/medizinische Kunststoffe/Varia.
Vermischungen dieser einzelnen Stoffe untereinander können das Wirkspektrum wesentlich erweitern und so eine Vielzahl von Infektionsmöglichkeiten absichern. Das Verfahren eignet sich aber auch dazu die primär vorgesehen Wirkstoffschicht zum Infektionsschutz als Carrier zu benutzen. So können z.B. dem entzündungshemmenden Wirkstoff, der die harte Wirkstoffschicht bildet, Wirkstoffe zur Bekämpfung von Krankheiten beigemengt werden. Dies sind z.B. die Zytostatika zur aktiven Bekämpfung von wuchernden Zellen. Insbesondere und erfindungsgemäß können an oder in die Wirkstoffschicht auch Stoffe beigemengt oder angeheftet werden, die im wesentlichen dazu geeignet sind, spezifische biologische Vorgänge zu beeinflussen. Im Falle der Knochenanbindung von Implantaten sind dies insbesondere Stoffe wie BMP (bone morphogenic proteins) oder FGF (fibrogen growth factors) oder genetisch manipulierte oder erzeugte Stoffe die das Knochenwachstum positiv beeinflussen. Diese Anlagerung/Einlagerung derartiger Stoffe ohne die Verwendung von Trägermaterialien hat den besonderen und erfindungsgemäßen Charakter der wirkstoffgebundenen Substanzeinbringung. Hervorzuheben ist dabei die freie Entfaltbarkeit der Wirkung der zellulär eingreifenden Substanzen ohne die hindernden Wirkung der sekundären Trägermaterialien, sowie den durch die eigentliche prophylaktische Wirkstoffschicht hervorgerufenen Wirkstoffschutz, der auch die Stoffe wie die Wachstumsfaktoren (z.B. BMP, FGF) und andere vor Infektionsbesiedelung schützt.

Es hat sich gezeigt, daß das Verfahren auch zur Beschichtung von Knochenersatzmaterialien geeignet ist. So konnte bei der Beschichtung von porösen keramischen Implantaten dieselbe harte, graduierte, einschichtige oder mehrschichtige Wirkstoffschicht aufgebracht werden. Die bekanntlich spontane Freisetzung von Wirkstoffen, insbesondere von Antibiotika, aus solchen Materialien konnte signifikant verbessert werden, so daß die Wirkung des Verfahrens dieselbe wie an der Oberfläche von Prothesen war. Insbesondere konnten Knochenersatzwerkstoffe auf der Basis von Calcium und Phosphat, von Calcium und Carbonat und Calcium und Sulfat natürlichen oder synthetischen Ursprunges verarbeitet werden. Dabei konnten Materialien sowohl keramischer Zusammensetzung als auch Materialien mit organischen Bestandteilen erfolgreich beschichtet werden.

Gleiches zeigte sich auch, und wird erfindungsgemäß beansprucht, bei der Beschichtung von Kunststoffen wie bei Inleays für Prothesen, Platten, Unterlegscheiben, Drainagen, Spritzenteilen, Keilen, Gazen und Häuten.
Aus der Vielzahl der beschichteten Werkstoffe wird geschlossen, daß auch andere Werkstoffe mit dieser Methode beschichtet werden können. Deshalb wird die Beschichtung von Werkstoffen wie Glaskeramik, Glas, Metallkeramik, Keramik und die Übergangswerkstoffe erfindungsgemäß beansprucht. Letztendlich kann das Verfahren auch zur Beschichtung von Schmuckteilen (Ohrhänger, Piercing-Teilen o.ä.) verwendet werden.

Die Erfindung soll nachstehend anhand von bevorzugten Ausführungsbeispielen und unter Bezugnahme auf die beigefügten Zeichnungen näher erläutert werden, wobei sich in den einzelnen Zeichnungen gleiche Bezugszeichen auf gleiche oder ähnliche Bestandteile beziehen.
Es zeigen:
- Fig. 1: eine erste Ausführungsform einer Vorrichtung zur erfindungsgemäßen Beschichtung eines Gegenstands mit einem Wirkstoff, und
- Fig. 2: eine weitere Ausführungsform der Vorrichtung zur erfindungsgemäßen Beschichtung eines Gegenstands mit einem Wirkstoff.

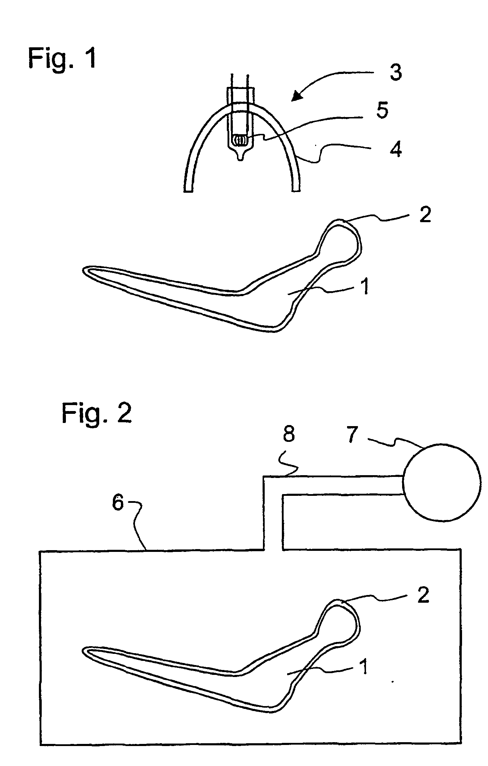

In Figur 1 ist eine erste Ausführungsform einer Vorrichtung zur Beschichtung mit einem Wirkstoff gezeigt. Bei den Gegenständen handelt es sich insbesondere um medizinische Implantate oder Onplantate. Bei dem in Fig. 1 gezeigten Implantat 1 handelt es sich beispielhaft um ein Hüftgelenkimplantat. Auf das Implantat 1 wird zunächst vermittels einer nicht gezeigten Einrichtung das Implantat 1 mit einer Schicht versehen, welche einen oder mehrere Wirkstoffe enthält. Die Schicht kann dabei eine Lösung der Wirkstoffe in einem geeigneten Lösungsmittel, wie etwa Wasser oder Alkohol sein. Das so vorbehandelte Implantat wird daraufhin mittels einer Strahlungsquelle 3 mit elektromagnetischen Wellen bestrahlt. Im Falle der in Fig. 1 gezeigten Ausführungsform ist die Strahlungsquelle eine Lichtquelle, die einen Strahlungserzeuger in Form einer Glühwendel 5 umfaßt. Die von der Glühwendel emittierte Strahlung fällt sowohl direkt, als auch reflektiert über den Reflektor 4 auf das mit der Schicht 2 beschichtete Implantat 1.

Der Reflektor 4 kann dazu ausgelegt sein, das von der Wendel 5 emittierte Strahlungsspektrum zu beeinflussen. Beispielsweise kann der Reflektor 4 durch Beschichtung mit. einer geeigneten Interferenzschicht als Kaltlichtreflektor ausgelegt sein, so daß insbesondere die kurzwelligen Anteile auf das Implantat fallen.

In Fig. 2 ist eine weitere Ausführungsform einer Vorrichtung. zur Beschichtung mit einem Wirkstoff gezeigt. Hier wird das Implantat 1, welches mit einer Schicht 2 aus in einer Lösung gelösten Wirkstoffen beschichtet ist, in ein metallisch leitendes Gehäuse 6 eingebracht, in welches über einen Hohlleiter 8 zum Beispiel Mikrowellen für die Behandlung des Implantats, bzw. für die Herstellung der Wirkstoffschicht eingestrahlt werden. Die elektromagnetischen Wellen werden von einer Quelle 7 erzeugt. Als Quelle 7 kann beispielsweise ein elektromagnetischer Generator verwendet werden.

Durch die Behandlung mittels den Strahlungsquellen der anhand der Figuren 1 und 2 beschriebenen beispielhaften Ausführungsformen für Vorrichtungen zur Beschichtung von Implantaten wird schließlich eine Wirkstoffschicht ohne sekundäre Trägermaterialien auf dem Implantat erzeugt.

## Patentansprüche

1. Vorrichtung zur Beschichtung eines Gegenstands mit einem Wirkstoff, umfassend :
eine erste Einrichtung, die mindestens einen Wirkstoff bereithält, eine weitere Einrichtung zum Auftragen.des Wirkstoffs auf den Gegenstand und eine Strahlungseinrichtung, **dadurch gekennzeichnet, daß** die energetische Strahlung der Strahlungseinrichtung auf den aufgebrachten Wirkstoff gerichtet ist, um den Gegenstand mit dem Wirkstoff unmittelbar zu beschichten, wobei die Strahlung eine kristalline oder eine amorphe Strukturschichtbildung des Wirkstoffes auf dem Gegenstand bewirkt.

2. Vorrichtung nach Anspruch 1,
**dadurch gekennzeichnet,**
**daß** die Strahlungseinrichtung elektromagnetische Strahlung erzeugt.

3. Vorrichtung nach einem der Ansprüche 1 oder 2,
**dadurch gekennzeichnet,**
**daß** die Strahlungseinrichtung, Strahlung mit gleichen oder unterschiedlichen Formen, insbesondere intermittierend oder gebündelt erzeugt.

4. Vorrichtung nach einem der vorstehenden Ansprüche,
**dadurch gekennzeichnet,**
**daß** die erste Einrichtung eine Mischung aus mindestens zwei Wirkstoffen bereithält.

5. Vorrichtung nach einem der vorstehenden Ansprüche,
**dadurch gekennzeichnet,**
**daß** die erste Einrichtung ein Gemisch aus mindestens einem Wirkstoff oder einer Wirkstoffmischung nach Anspruch 4 und einem anderen Stoff bereithält, insbesondere in einer Lösung oder Suspension.

6. Gemisch für eine Vorrichtung nach Anspruch 5,
**dadurch gekennzeichnet, daß** der andere Stoff Wasser oder Alkohol ist, insbesondere bidestilliertes steriles Wasser.

7. Vorrichtung nach einem der Ansprüche 4 bis 6,
**dadurch gekennzeichnet,**
**daß** die weitere Einrichtung zum Auftragen der Wirkstoffmischung oder des Gemischs dient.

8. Vorrichtung nach einem der vorstehenden Ansprüche,
**dadurch gekennzeichnet,**
**daß** der Wirkstoff eine der folgenden Substanzen oder eine Kombination von folgenden Substanzen umfaßt :
Abmagerungsmittel/Appetitzügler, Analeptika /Antihypoxämika, Analgetika/Antirheumatika, Antiallergika, Antianämika, Antiarrhythmika, Antibiotika/Antiinfektiva, Antidementiva, Nootropika, Antidiabetika, Antidota, Antiemetika/Antivertiginosa, Antiepileptika, Antihämorrhagika, Antifibrinolytika, Hämostatika, Antihypertonika, Antihypoglykämika, Antihypotonika, Antikoagulantia, Antimykotika, Antiparasitäre Mittel, Antiphlogistika, Antitussiva/Expektorantia, Arteriosklerosemittel, Balneotherapeutika u. Mittel zur Wärmetherapie, Betarezeptorenblocker, Calciumkanalblocker u. Hemmstoffe des Renin-Angiotensin-System, Broncholytika /Antiasthmatika, Clindamycine, Cholagoga u. Gallenwegstherapeutika, Cholinergika, Corticoide, Dermatika, Desinfizientia/Antiseptika, Diätetika /Ernährungstherapeutika, Diagnostika u. Mittel zur Diagnosevorbereitung, Diuretika, Durchblutungsfördernde Mittel, Entwöhnungsmittel, Enzyminhibitoren, Enzympräparate u. Transportproteine, Fibrinolytika, Gentamicinsulfat, Geriatrika, Gichtmittel, Grippemittel u. Mittel gegen Erkältungskrankheiten, Gynäkologika, Hepatika, Hypnotika/Sedativa, Hypophysen-, Hypothalamushormone, andere regulatorische Peptide u. ihre Hemmstoffe, Immunmodulatoren, Infusions- u. Standardinjektionslösungen, Organperfusionslösungen, Kardiaka, Karies-, Paradontosemittel u. andere Dentalpräparate, Koronarmittel, Laxantia, Lipidsenker, Lokalanästhetika/Neuraltherapeutika, Magen-Darm-Mittel, Migränemittel, Mineralstoffpräparate, Mund- u. Rachentherapeutika, Muskelrelaxantia, Narkosemittel, Neuropathiepräparate u. andere neurotrope Mittel, Ophthalmika, Osteoporosemittel/Calciumstoffwechselregulatoren, Otologika, Parkinsonmittel u. andere Mittel gegen extrapyramidale Störungen, Penicillin, Psychopharmaka, Rhinologika/Sinusitismittel, Roborantia/Tonika, Schilddrüsentherapeutika, Sera, Immunglobuline u. Impfstoffe, Sexualhormone u. ihre Hemmstoffe, Spasmolytika, Thrombozytenaggregationshemmer, Tuberkulosemittel, Umstimmungsmittel, Urologika, Vancomycine, Venentherapeutika, Vitamine, Wundbehandlungsmittel, Zytostatika, andere antineoplastische Mittel u. Protektiva, Präparateserien/Reg. Homöopathika oder Biomaterialien / medizinische Kunststoffe , Wachstumsfaktoren.

9. Verfahren zur Beschichtung eines Gegenstands mit einem Wirkstoff, umfassend :
a) Aufbringen mindestens eines Wirkstoffs auf den Gegenstand
und
b) Bestrahlen des aufgebrachten Wirkstoffs, **dadurch gekennzeichnet, daß** das Bestrahlen des aufgebrachten Wirkstoffs den Gegenstand unmittelbar ohne sekundäre Trägermaterialien beschichtet, wobei durch das Bestrahlen eine kristalline oder eine amorphe Strukturschichtbildung des Wirkstoffes auf dem Gegenstand bewirkt wird.

10. Verfahren zur Beschichtung eines Gegenstands mit einem Wirkstoff,
**dadurch gekennzeichnet, daß**
a) mindestens ein Wirkstoff mit sekundärem Trägermaterial auf den Gegenstand aufgebracht wird und
b) die Bestrahlung des mit sekundärem Trägermaterial aufgebrachten Wirkstoffs vorgenommen wird, wobei
durch das Bestrahlen eine kristalline oder eine amorphe Strukturschichtbildung des Wirkstoffes auf dem Gegenstand bewirkt wird.

11. Verfahren nach Anspruch 9 oder 10,
**dadurch gekennzeichnet,**
**daß** der Wirkstoff eine der unter Anspruch 8 beschriebenen Substanzen oder eine Kombination von diesen Substanzen umfaßt.

12. Verfahren nach einem der Ansprüche 9 bis 11,
**dadurch gekennzeichnet,**
**daß** eine Mischung aus mindestens zwei Wirkstoffen auf den Gegenstand aufgebracht wird.

13. Verfahren nach einem der Anspruch 9 bis 12,
**dadurch gekennzeichnet,**
**daß** der Wirkstoff oder die Wirkstoffmischung nach Anspruch 11 mit einem anderen Stoff zu einer Lösung oder Suspension gemischt wird.

14. Verfahren nach Anspruch 13,
**dadurch gekennzeichnet,**
**daß** der andere Stoff, Alkohol oder Wasser oder ein anderes Lösungsmittel, temporär vorhanden ist, insbesondere bidestilliertes Wasser.

15. Verfahren nach einem der Ansprüche 13 oder 14,
**dadurch gekennzeichnet,**
**daß** die Lösung oder Suspension oder Emulsion auf den Gegenstand aufgebracht wird.

16. Verfahren nach einem der Ansprüche 9 bis 15,
**dadurch gekennzeichnet,**
**daß** die aufgebrachte Wirkstoffmischung oder die Lösung oder die Suspension bestrahlt wird.

17. Verfahren nach einem der Ansprüche 9 bis 16,
**dadurch gekennzeichnet,**
**daß** das Bestrahlen durch eine energetische Strahlung geschieht.

18. Verfahren nach einem der Ansprüche 9 bis 17,
**dadurch gekennzeichnet,**
**daß** das Bestrahlen durch eine elektromagnetische Strahlung erfolgt.

19. Verfahren nach einem der Ansprüche 9 bis 18,
**dadurch gekennzeichnet,**
**daß** die Bestrahlung kontinuierlich oder diskontinuierlich bei einem oder mehreren Frequenzbereichen erfolgt.

20. Verfahren nach einem der Ansprüche 9 bis 19,
**dadurch gekennzeichnet,**
**daß** die Strahlung nur zu einem geringen Teil eine Erwärmung der aufgebrachten Flüssigkeitsschicht bewirkt.

21. Verfahren nach einem der Ansprüche 9 bis 20,
**dadurch gekennzeichnet, daß**
durch die Strahlung eine kristalline oder eine amorphe Strukturschichtbildung der Wirkstoffe oder der Wirkstoffmischung nach Anspruch 4 oder 5 auf dem Gegenstand bewirkt wird.

22. Verfahren nach einem der Ansprüche 9 bis 21,
**dadurch gekennzeichnet,**
**daß** der Gegenstand vorbehandelt wird, insbesondere die Oberfläche vergrößert und/oder die Oberfläche elektrisch aufgeladen ist, so daß dieser Gegenstand Flüssigkeiten vollflächig aufnehmen kann.

23. Verfahren nach einem der Ansprüche 9 bis 22,
**dadurch gekennzeichnet,**
**daß** man den zu beschichtenden Gegenstand oder ein Teil davon vorbehandelt, insbesondere von gegenstandsfremden Substanzen säubert.

24. Verfahren nach einem der Ansprüche 9 bis 23,
**dadurch gekennzeichnet,**
**daß** der Gegenstand oder Teile davon unterschiedlich oder gleich dick mit dem Wirkstoff beschichtet werden.

25. Verfahren nach einem der Ansprüche 9 bis 24,
**dadurch gekennzeichnet,**
**daß** die Schichtdicke im Nanometer - Bereich bis in den Millimeter - Bereich liegen kann.

26. Verfahren nach einem der Ansprüche 9 bis 25,
**dadurch gekennzeichnet,**
**daß** der Gegenstand
- mit einer Lage oder mehreren Lagen,
- mit verschiedenen oder gleichen Wirkstoffen,
- mit organischen Bestandteilen,
- in Verbindung mit zellulär wirksamen Bestandteilen,
- in wirkstoffrelevanten Abständen,
- mindestens ein Teil der Oberfläche,
- in Segmenten einseitig oder mehrseitig,
- verschiedene Teile der Oberfläche mit unterschiedlichen Wirkstoffen
und/oder
- auf seiner ganzen Oberfläche beschichtet wird.

27. Verfahren nach einem der Ansprüche 9 bis 26,
**dadurch gekennzeichnet,**
**daß** die Wirkstoffschichten gleich oder unterschiedlich löslich aufgebracht werden, insbesondere eine graduierte Freisetzungsrate des oder der Wirkstoffe beinhaltet.

28. Verfahren nach einem der Ansprüche 9 bis 27,
**dadurch gekennzeichnet,**
**daß** die Beschichtung einen arzneilich wirksamen/pharmakologisch wirksamen/pharmazeutisch wirksamen Bestandteil enthält.

29. Verfahren nach einem der Ansprüche 9 bis 28,
**dadurch gekennzeichnet,**
**daß** die Beschichtung in ihrer Härte graduiert und/oder in ihrer mechanischen Härte einstellbar ist, insbesondere eine feste Schicht, eine elastische Schicht oder eine weiche Schicht.

30. Verfahren nach einem der Ansprüche 9 bis 29,
**dadurch gekennzeichnet,**
**daß** durch die Energiedosis der Härtegrad der Beschichtung bestimmt wird, insbesondere mit einer höheren Dosis eine härtere Beschichtung und mit einer geringeren Dosis eine weichere Beschichtung.

31. Verfahren nach einem der Ansprüche 9 bis 30,
**dadurch gekennzeichnet,**
**daß** die Beschichtung eine Kennzeichenfärbung enthält.

32. Verfahren nach einem der Ansprüche 9 bis 31,
**dadurch gekennzeichnet,**
**daß** andere Wirkstoffe an die Beschichtung angelagert und/oder eingebettet sind.

33. Verfahren nach Anspruch 32,
**dadurch gekennzeichnet,**
**daß** der andere Wirkstoff'ein Wachstumsfaktor oder Wachstumsbeschleuniger ist.

34. Verwendung des Verfahrens nach einem der Ansprüche 9 bis 33 für die Beschichtung von
- einem Implantat, insbesondere ein Hüft -, Knie-, Schulter-, oder Kieferimplantat, chirurgisches oder orthopädisches Implantat,
- einem Onplantat, insbesondere chirurgisches oder orthopädisches Onplantat,
- einem Inlay oder Onlay,
- einem medizinischen Hilfsmittel, insbesondere Nagel, Schraube, Draht, Fixateure, Hülse, Kanüle, Nadel, Nahtmaterial, Klammer oder Drainage,
- einem medizinischen Instrument,
- einem medizinischen Produkt, insbesondere invasive oder aktive Medizinprodukte,
- einem medizinischen Gerät, insbesondere Pumpen, Dosiergeräte, Signalgeräte, Impulsgeber oder Insulindetektoren,
- einer Sehhilfe oder Hörhilfe,
- einer Kriochenersatzkeramik, insbesondere auf Basis von Calcium und Phosphat,
- einer Knochenersatzmaterial, insbesondere auf Basis von Keramik, Calcium und Carbonat oder Calcium und Sulfat,
- einem Band, einer Sehne oder Platte,
- einem Gegenstand aus Glas, Keramik, Kunststoff, Metall, Glaskeramik oder Metallkeramik,
oder
- einem Schmuckteil.

35. Erzeugnis hergestellt durch das Verfahren nach einem der Ansprüche 9 bis 34.

## Claims

1. A device for coating an object with an active substance, comprising:
a first device, which keeps at least one active substance ready for use,
a further device for applying the active substance onto the object, and
a radiation device, **characterised in that** the energy radiation of said radiation device is directed onto the active substance applied in order to coat the object directly with the active substance, the radiation causing a crystalline or amorphous structure coating formation of the active substance on the object.

2. The device as recited in Claim 1,
wherein the radiation device produces electromagnetic radiation.

3. The device as recited in one of Claims 1 or 2,
wherein the radiation device produces radiation having identical or different forms, in particular intermittently or bundled.

4. The device as recited in one of the preceding claims,
wherein the first device keeps a mixture of at least two active substances ready for use.

5. The device as recited in one of the preceding claims,
wherein the first device keeps a mixture of at least one active substance or an active substance mixture as recited in Claim 4 and another material ready, in particular in a solution or suspension.

6. A mixture for a device as recited in Claim 5,
wherein the other material is water or alcohol, in particular double-distilled sterile water.

7. The device as recited in one of Claims 4 through 6,
wherein the further device is used for applying the active substance mixture or the mixture.

8. The device as recited in one of the preceding claims,
wherein the active substance includes one of the following substances or a combination of the following substances:
weight reducing agents/appetite suppressants, analeptics/antihypoxemics, analgesics/antirheumatics, antiallergics, antianemics, antiarrhythmics, antibiotics/antiinfectants, antidementia drugs, nootropics, antidiabetics, antidotes, antiemetics/antivertigo drugs, antiepileptics, antihemorrhagics, antifibrinolytics, hemostatics, antihypertensives, antihypoglycemics, antihypotensives, anticoagulants, antimycotics, antiparasitic agents, antiphlogistics, antitussives/expectorants, arteriosclerosis agents, balneotherapeutics and agents for heat therapy, beta receptor blockers, calcium channel blockers and inhibitors of the renin-angiotensin system, bronchiolytics/antiasthmatics, cholagogues and gallbladder pharmaceuticals, cholinergics, corticoids, dermatics, disinfectants/antiseptics, dietetic agents/nutrition treatments, diagnostics and agents for diagnosis preparation, diuretics, circulatory enhancement agents, withdrawal agents, enzyme inhibitors, enzyme preparations and transport proteins, fibrinolytics, geriatric agents, gout agents, anti-influenza agents and agents against colds, gynecological agents, hepatic agents, hypnotics/sedatives, pituitary hormones, hypothalamus hormones, other regulatory peptides and their inhibitors, immune modulators, infusion and standard injection solutions, organ perfusion solutions, cardiac drugs, caries agents, periodontosis agents and other dental preparations, coronary agents, laxatives, antilipemics, local anesthetics/neurotherapeutics, gastrointestinal agents, migraine agents, mineral preparations, oropharyngeal treatments, muscle relaxants, narcotics, neuropathy preparations and other neurotropic agents, ophthalmics, osteoporosis agents/calcium metabolism regulators, otologics, Parkinson's agents and other agents against extrapyramidal disorders, psychopharmaceuticals, rhinologic agents/sinusitis agents, reconstituents/tonics, thyroid treatments, serums, immunoglobulin and vaccines, sexual hormones and their inhibitors, spasmolytics, thrombocyte aggregation inhibitors, tuberculosis agents, immune enhancers, urologics, venous treatments, vitamins, wound treatment agents, cytostatics, other antineoplastic agents and protectants, prepared serums/registered homeopathics, biomaterials/medical plastics, or growth factors.

9. A method of coating an object with an active substance, including:
a) applying at least one active substance to the object and
b) irradiating the active substance applied **characterised in that** the object is coated directly without secondary carrier materials by irradiation the active substance applied, the radiation causing a crystalline or amorphous structure coating formation of the active substance on the object.

10. A method of coating an object with an active substance,
wherein
a) at least one active substance is applied to the object using secondary carrier material and
b) the active substance applied using secondary carrier material is irradiated, the radiation causing a crystalline or amorphous structure coating formation of the active substance on the object.

11. The method as recited in Claim 9 or 10,
wherein the active substance includes one of the substances described in Claim 8 or a combination of these substances.

12. The method as recited in one of Claims 9 through 11,
wherein a mixture of at least two active substances is applied to the object.

13. The method as recited in one of Claims 9 through 12,
wherein the active substance or the active substance mixture as recited in Claim 11 is mixed with another material to form a solution or suspension.

14. The method as recited in Claim 13,
wherein the other material, alcohol or water or another solvent, in particular double-distilled water, is present temporarily.

15. The method as recited in one of Claims 13 or 14,
wherein the solution or suspension or emulsion is applied to the object.

16. The method as recited in one of Claims 9 through 15,
wherein the active substance mixture or the solution or the suspension applied is irradiated.

17. The method as recited in one of Claims 9 through 16,
wherein the irradiation is performed through energetic radiation.

18. The method as recited in one of Claims 9 through 17,
wherein the irradiation is performed through electromagnetic radiation.

19. The method as recited in one of Claims 9 through 18,
wherein the irradiation is performed continuously or discontinuously in one or more frequency ranges.

20. The method as recited in one of Claims 9 through 19,
wherein the radiation causes only slight heating of the liquid coating applied.

21. The method as recited in one of Claims 9 through 20,
wherein the radiation causes the formation of a crystalline or amorphous coating structure of the active substances, or the active substance mixture as recited in Claim 4 or 5 on the object.

22. The method as recited in one of Claims 9 through 21,
wherein the object is pretreated, in particular the surface is enlarged and/or the surface is electrically charged, so that this object may absorb liquids over its entire surface area.

23. The method as recited in one of Claims 9 through 22,
wherein the object to be coated or a part thereof is pretreated, and in particular is cleaned of substances foreign to the object.

24. The method as recited in one of Claims 9 through 23,
wherein the object or parts thereof are coated with a layer of the active substance having different or identical thicknesses.

25. The method as recited in one of Claims 9 through 24,
wherein the coating thickness may lie in the nanometer range up to the millimeter range.

26. The method as recited in one of Claims 9 through 25,
wherein the object is coated
- using one layer or multiple layers,
- using different or identical active substances,
- using organic components,
- in combination with components having cellular activity,
- using spacings relevant for the active substance,
- on at least a part of the surface,
- in segments on one side or multiple sides,
- on different parts of the surface using different active substances, and/or
- on its entire surface.

27. The method as recited in one of Claims 9 through 26,
wherein the active substance coatings may be applied with identical or different solubilities, and in particular include a graduated release rate of the active substance(s).

28. The method as recited in one of Claims 9 through 27,
wherein the coating contains a medicinally active/pharmacologically active/pharmaceutically active component.

29. The method as recited in one of Claims 9 through 28,
wherein the coating is graduated in its hardness and/or adjustable in its mechanical hardness, and in particular is a hard coating, an elastic coating, or a soft coating.

30. The method as recited in one of Claims 9 through 29,
wherein the degree of hardness of the coating is determined through the energy dose, in particular using a higher dose for a harder coating and using a lower dose for a softer coating.

31. The method as recited in one of Claims 9 through 30 ,
wherein the coating contains an identifying color.

32. The method as recited in one of Claims 9 through 31,
wherein other active substances are attached onto and/or embedded into the coating.

33. The method as recited in Claim 32,
wherein the other active substance is a growth factor or growth accelerator.

34. Use of the method as recited in one of Claims 9 through 33 for coating
- an implant, in particular a hip, knee, shoulder, or jaw implant, surgical implant, or orthopedic implant,
- an onplant, in particular a surgical or orthopedic onplant,
- an inlay or onlay,
- a medical aid, in particular nails, screws, wires, fixators, sleeves, cannulas, needles, suture material, clamps, or drains,
- a medical instrument,
- a medical product, in particular invasive or active medical products,
- a medical device, in particular pumps, metering devices, signaling devices, pulse generators, or insulin detectors,
- a vision aid or hearing aid,
- a bone replacement ceramic, in particular based on calcium and phosphate,
- a bone replacement material, in particular based on ceramic, calcium and carbonate, or calcium and sulfate,
- a ligament, a tendon, or a plate,
- an object made of glass, ceramic, plastic, metal, glass ceramic, or metal ceramic, or
- a jewelry part.

35. A product manufactured by the method as recited in one of Claims 9 through 34.

## Revendications

1. Dispositif destiné au revêtement d'un objet avec un principe actif, comportant :
un premier appareil, qui tient à disposition au moins un principe actif, un autre appareil pour l'application du principe actif sur l'objet, et un appareil de rayonnement,
**caractérisé en ce que** le rayonnement énergétique de l'appareil de rayonnement est dirigé sur le principe actif appliqué afin de revêtir l'objet directement avec le principe actif, le rayonnement engendrant sur l'objet une formation de couche à structure cristalline ou amorphe du principe actif.

2. Dispositif selon la revendication 1,
**caractérisé en ce que** l'appareil de rayonnement génère un rayonnement électromagnétique.

3. Dispositif selon l'une des revendications 1 ou 2,
**caractérisé en ce que** l'appareil de rayonnement génère un rayonnement de formes identiques ou différentes, notamment un rayonnement intermittent ou focalisé.

4. Dispositif selon l'une quelconque des revendications précédentes,
**caractérisé en ce que** le premier appareil tient à disposition un mélange constitué d'au moins deux principes actifs.

5. Dispositif selon l'une quelconque des revendications précédentes,
**caractérisé en ce que** le premier appareil tient à disposition un mélange constitué d'au moins un principe actif, ou d'un mélange de principes actifs selon la revendication 4 et d'une autre substance, notamment en une solution ou suspension.

6. Mélange pour un dispositif selon la revendication 5,
**caractérisé en ce que** l'autre substance est de l'eau ou de l'alcool, notamment de l'eau stérile bidistillée.

7. Dispositif selon l'une quelconque des revendications 4 à 6,
**caractérisé en ce que** l'autre appareil sert à l'application du mélange de principes actifs ou du mélange.

8. Dispositif suivant l'une des revendications précédentes, **caractérisé en ce que** le principe actif comprend l'une des substances suivantes ou une combinaison de substances suivantes :
agents amaigrissants/anorexiants, analeptiques/antihypoxémiques, analgésiques/antirhumatismaux, antiallergiques, antianémiques, antiarhytmiques, antibiotiques/anti-infectieux, agents anti-démence, produits nootropes, antidiabétiques, antidotes, antiémétiques/produits contre les vertiges, antiépileptiques, antihémorragiques, antifibrinolytiques, hémostatiques, antihypertoniques, antihypoglycémiques, antihypotoniques, anticoagulants, antimycosiques, agents antiparasitaires, antiphlogistiques, antitussifs/expectorants, agents anti-artériosclérose, produits balnéothérapiques et produits pour la thermothérapie, inhibiteurs des récepteurs β-adrénergiques, antagonistes du calcium et inhibiteurs du système rénine-angiotensine, broncholytiques/antiasthmatiques, clindamycine, cholagogues et médicaments pour les voies biliaires, cholinergiques, corticoïdes, produits dermatologiques, désinfectants/anti-septiques, produits diététiques/produits de thérapie nutritionnelle, produits de diagnostic et produits de préparation en vue du diagnostic, diurétiques, agents favorisant l'irrigation sanguine, produits de désintoxication, inhibiteurs d'enzymes, préparations enzymatiques et protéines de transfert, agents fibrinolytiques, sulfate de gentamicine, produits gériatriques, produits antigoutteux, agents antigrippaux et produits contre des affections causées par un refroidissement, produits gynécologiques, produits contre les maladies hépatiques, somnifères/sédatifs, hormone hypophysaire, hormone hypothalamique, autres peptides régulateurs et leurs inhibiteurs, immunomodulateurs, solutions pour perfusion et solutions injectables standard, solutions pour la perfusion d'organes, produits de cardiologie, agents anti-carie, produits contre la parondotose et autres préparations dentaires, agents coronariens, laxatifs, hypolipidémiants, anesthésiques locaux/agents neurothérapiques, produits pour gastroentérologie, agents antimigraineux, préparations de substances minérales, médicaments buccaux et pharyngiens, myorelaxants, anesthésiques, préparations neuropathiques et autres agents neurotropes, produits à usage ophtalmique, agents contre l'ostéoporose/régulateurs du métabolisme calcique, produits pour l'otologie, antiparkinsoniens et autres agents contre les troubles extrapyramidaux, pénicilline, médicaments psychotropes, produits pour rhinologie/agents contre la sinusite, fortifiants/toniques, produits thyroïdothérapiques, sérums, immunoglobulines et vaccins, hormones sexuelles et leurs inhibiteurs, agents spasmolytiques, inhibiteurs d'agrégation plaquettaire, produits antituberculeux, produits pour la modification de fonctions physiologiques, produits urologiques, vancomycines, produits de thérapie veineuse, vitamines, produits pour le traitement des plaies, cytostatiques, autres agents antinéoplasiques et produits protecteurs, séries de préparations/produits homéopathiques courants ou biomatériaux/matières synthétiques à usage médical, facteurs de croissance.

9. Procédé de revêtement d'un objet avec un principe actif, comportant :
a) l'application d'au moins un principe actif sur l'objet, et
b) l'exposition au rayonnement du principe actif appliqué, **caractérisé en ce que** l'exposition au rayonnement du principe actif appliqué assure le revêtement direct de l'objet sans substances de support secondaires, le rayonnement engendrant sur l'objet une formation de couche à structure cristalline ou amorphe du principe actif.

10. Procédé de revêtement d'un objet avec un principe actif,
**caractérisé en ce que**
a) au moins un principe actif contenant une substance de support secondaire est appliqué sur l'objet, et
b) le principe actif appliqué avec la substance de support secondaire est exposé au rayonnement,
le rayonnement engendrant sur l'objet une formation de couche à structure cristalline ou amorphe du principe actif.

11. Procédé selon la revendication 9 ou 10,
**caractérisé en ce que** le principe actif contient une des substances décrites dans la revendication 8, ou une combinaison de ces substances.

12. Procédé selon l'une quelconque des revendications 9 à 11,
**caractérisé en ce qu'**un mélange d'au moins deux principes actifs est appliqué sur l'objet.

13. Procédé selon l'une quelconque des revendications 9 à 12,
**caractérisé en ce que** le principe actif ou le mélange de principes actifs selon la revendication 11 est mélangé avec une autre substance en une solution ou suspension.

14. Procédé de revêtement selon la revendication 13,
**caractérisé en ce que** l'autre substance, de l'alcool ou de l'eau ou un autre solvant, est présente temporairement, notamment de l'eau bidistillée.

15. Procédé selon l'une des revendications 13 ou 14,
**caractérisé en ce que** la solution ou la suspension ou l'émulsion est appliquée sur l'objet.

16. Procédé selon l'une quelconque des revendications 9 à 15,
**caractérisé en ce que** le mélange de principes actifs appliqué, ou la solution ou la suspension, est exposé au rayonnement.

17. Procédé selon l'une quelconque des revendications 9 à 16,
**caractérisé en ce que** l'exposition au rayonnement est effectuée par un rayonnement énergétique.

18. Procédé selon l'une quelconque des revendications 9 à 17,
**caractérisé en ce que** l'exposition au rayonnement est effectuée par un rayonnement électromagnétique.

19. Procédé selon l'une quelconque des revendications 9 à 18,
**caractérisé en ce que** l'exposition au rayonnement est effectuée en continu ou en discontinu dans une ou plusieurs plages de fréquence.

20. Procédé selon l'une quelconque des revendications 9 à 19,
**caractérisé en ce que** l'exposition au rayonnement ne provoque que dans une faible mesure un réchauffement de la couche de liquide appliquée.

21. Procédé selon l'une quelconque des revendications 9 à 20,
**caractérisé en ce que** le rayonnement engendre sur l'objet une formation de couche à structure cristalline ou amorphe des principes actifs ou du mélange de principes actifs selon la revendication 4 ou 5.

22. Procédé selon l'une quelconque des revendications 9 à 21,
**caractérisé en ce que** l'objet est préalablement traité, la surface étant notamment agrandie et/ou la surface étant chargée électriquement, de sorte que cet objet puisse absorber des liquides sur toute sa surface.

23. Procédé selon l'une quelconque des revendications 9 à 22,
**caractérisé en ce que** l'objet à revêtir ou une partie de celui-ci est soumis à un traitement préalable, notamment débarrassé de substances étrangères.

24. Procédé selon l'une quelconque des revendications 9 à 23,
**caractérisé en ce que** l'objet ou des parties de celui-ci est revêtu par le principe actif avec différentes épaisseurs ou la même épaisseur.

25. Procédé selon l'une quelconque des revendications 9 à 24,
**caractérisé en ce que** l'épaisseur de couche peut se situer dans la plage des nanomètres jusque dans la plage des millimètres.

26. Procédé selon l'une quelconque des revendications 9 à 25,
**caractérisé en ce que**
l'objet est revêtu
- d'une ou de plusieurs couches,
- avec des principes actifs différents ou identiques,
- avec des composés organiques,
- en liaison avec des composés agissant sur les cellules,
- à des écartements importants pour les principes actifs,
- sur une partie au moins de sa surface,
- en segments sur une ou plusieurs faces,
- sur différentes parties de la surface avec différents principes actifs,
et/ou
- sur l'ensemble de sa surface.

27. Procédé selon l'une quelconque des revendications 9 à 26,
**caractérisé en ce que** les couches de principes actifs sont appliquées en étant solubles de façons identiques ou différentes, notamment **en ce qu'**elles présentent un taux de libération gradué du ou des principes actifs.

28. Procédé selon l'une quelconque des revendications 9 à 27,
**caractérisé en ce que** le revêtement contient un composé efficace en matière de thérapie / efficace en matière de pharmacologie / efficace en matière pharmaceutique.

29. Procédé selon l'une quelconque des revendications 9 à 28,
**caractérisé en ce que** le revêtement peut être gradué dans sa dureté et/ou réglé dans sa dureté mécanique, notamment en une couche dure, une couche élastique ou une couche souple.

30. Procédé selon l'une quelconque des revendications 9 à 29,
**caractérisé en ce que** le degré de dureté du revêtement est défini par la dose d'énergie, notamment **en ce qu'**un revêtement plus dur est défini par une dose plus forte, et un revêtement plus souple par une dose plus faible.

31. Procédé selon l'une quelconque des revendications 9 à 30,
**caractérisé en ce que** le revêtement contient une coloration d'identification.

32. Procédé selon l'une quelconque des revendications 9 à 31,
**caractérisé en ce que** d'autres principes actifs sont fixés sur et/ou enrobés dans le revêtement.

33. Procédé selon la revendication 32,
**caractérisé en ce que** l'autre principe actif est un facteur de croissance ou un accélérateur de croissance.

34. Utilisation du procédé selon l'une quelconque des revendications 9 à 33 pour le revêtement
- d'un implant, notamment d'un implant de hanche, de genou, d'épaule, ou d'un implant maxillaire, d'un implant chirurgical ou orthopédique,
- d'un onplant, notamment d'un onplant chirurgical ou orthopédique,
- d'un inlay ou onlay,
- d'un moyen auxiliaire médical, notamment d'un clou, d'une vis, d'un fil, de fixateurs, de douilles, de canules, d'aiguilles, de matériau de suture, d'agrafes ou de drainage,
- d'un instrument médical,
- d'un produit médical, notamment de produits médicaux invasifs ou actifs,
- d'un appareil médical, notamment de pompes, d'appareils de dosage, d'appareils de signalisation, d'impulseurs ou de détecteurs d'insuline,
- d'une prothèse visuelle ou auditive,
- d'une céramique de substitution d'os, notamment à base de calcium et de phosphate,
- d'un matériau de substitution d'os, notamment à base de céramique, de calcium et de carbonate, ou de calcium et de sulfate,
- d'une bande, d'un tendon ou d'une plaque,
- d'un objet en verre, en céramique, en matière plastique, en métal, en vitrocéramique ou en céramique métallique,
ou
- d'une pièce de bijou.

35. Produit fabriqué avec le procédé selon l'une quelconque des revendications 9 à 34.
